# EUROPEAN PATENT SPECIFICATION

(11) **EP 2 500 026 B1**
(45) Date of publication and mention of the grant of the patent: **22.05.2013**
(21) Application number: 11158593.1
(22) Date of filing: 17.03.2011
(51) Int. Cl.: A61K 36/328, A61K 9/00, A61P 15/12

(54) **Treatment of menopausal symptoms as novel indication for myrrh**
Behandlung von menopausalen Symptomen als neuartige Indikation für Myrrhe
Traitement des symptômes de la ménopause en tant que nouvelle indication pour myrrhe

(43) Date of publication of application: 19.09.2012
(73) Proprietor: Sonia Pharma GmbH, 35285 Gemünden/Wohra (DE)
(72) Inventor: Brandt, Eva, 35285, Gemünden/Wohra (DE)
(74) Representative: Sokolowski, Fabian

(56) References cited:
- WO-A1-98/42188
- DATABASE WPI Week 200626 Thomson Scientific, London, GB; AN 2006-241971 XP000002656178, & CN 1 679 687 A (YAO S) 12 October 2005 (2005-10-12)
- DATABASE WPI Week 200825 Thomson Scientific, London, GB; AN 2008-D29311 XP000002656179, & CN 101 057 892 A (HENAN WANXI PHARM CO LTD) 24 October 2007 (2007-10-24)
- DATABASE WPI Week 200526 Thomson Scientific, London, GB; AN 2005-243695 XP000002656180, & CN 1 559 586 A (CAO J) 5 January 2005 (2005-01-05)
- DATABASE WPI Week 200113 Thomson Scientific, London, GB; AN 2001-123342 XP000002656181, & ZA 200 000 192 A (JULLIENNE J R J) 27 September 2000 (2000-09-27)

## Description

The instant invention relates to a novel indication for myrrh according to claim 1.

### Myrrh Resin

Myrrh is the oleo-gum resin obtained from stems and branches of Commiphora molmol Engler and other related species of Commiphora other than Commiphora mukul. Commiphora mukul - although sharing the genus name - cannot be used for obtaining myrrh. Commiphora molmol Engler is sometimes also referred to as Commiphora myrrha or Commiphora myrrha (Nees) Engler. Suited related species of Commiphora molmol for obtaining myrrh are Commiphora abyssinica Engler and Commiphora schimperi Engler. Pharmacopoeia myrrh is generally obtained from Commiphora molmol Engler, but the other related species, in particular those referred to above, are also well suited.

For medical purposes, myrrh is used as powdered resin, capsules, myrrh tincture and other galenical preparations for topical use.

Myrrh can be separated into three components: volatile oil (ca. 2 to 10%), alcohol-soluble resin (ca. 25 to 40 %) and water-soluble gum.

The main constituents of myrrh essential oil are furanosesquiterpenes of various structural types together with sesquiterpenes. Furanosesquiterpenes are the source of its characteristic balsamic odour; a mixture of furanoeudesma-1,3-diene and lindestrene has the typical aroma of myrrh.

Myrrh essential oil is generally obtained by hydrodistillation, steam distillation, and solvent extraction. Extraction by carbon dioxide in the supercritical state is the state-of-the-art process that offers many advantages in obtaining volatile extracts. The mild extraction conditions give assurance against chemical reactions not taking place during the process; i.e. no hydrolysis, oxidation, or isomerisation taking place. Other possible extraction agents include nitrogen, hexane, methane, and ethane.

Marongiu et al.: "Chemical Composition of the Essential Oil and Supercritical CO2 Extract of Commiphora myrrha (Nees) Engl. and of Acorus calamus L.", J. Agric. Food Chem. 53 (2005), 7939-7943 discloses the composition of myrrh extract obtained by different methods. The authors demonstrated that hydrodistillation (HD), steam distillation (SD) and supercritical extraction with carbon dioxide (SFE) yield similar results as far as main components and quantities extracted are concerned.

Myrrh is known for its wound healing effect. Dolara, P. et al.: "Local Anaesthetic, Antibacterial and Antifungal Properties of Sesquiterpenes from Myrrh", Planta Medica 66 (2000), 356-358 describe the anaesthetic, antibacterial and antifungal properties of certain sesquiterpenes of myrrh.

US 4,719,111 A discloses the external use of myrrh gum for treating decubitus ulcers.

US 4,592,912 A discloses the topical use for the relief of or for the prevention of muscular aches, pains, cramps and muscular spasms such as are found, for example, in overexerted muscles, misused muscles, headaches and back aches.

US 5,350,774 A describes the topical treatment of skin disorders with myrrh.

US 5,248,503 A discloses the utilization of myrrh as food or dietary supplement.

US 6,077,513 A discloses a pharmaceutical composition containing myrrh oil and myrrh resin as the active ingredients therein for treating schistosomiasis.

### Physiology of Menopausal Vaginal Symptoms

The menopause is defined as the permanent cessation of cyclical menstruation due to loss of ovarian follicular activity. The decline in estrogen concentration is associated with both acute and long-term effects. Established acute symptoms are vasomotor instability, manifesting as hot flushes and night sweats, and vaginal atrophy, manifesting as vaginal dryness, itching, burning, and discomfort.

Menopausal symptoms may be managed by using hormone replacement therapy (HRT) with estrogens, with or without progestogens. Adverse effects of HRT include an increased risk of breast cancer, ovarian and endometrial cancer, an increased risk of venous thromboembolism, stroke, dementia, gallstone formation and cholecystectomy. It is now generally recommended that menopausal HRT used to relieve vasomotor and vaginal symptoms should be given at the lowest effective dose for no longer than necessary. But to withdraw HRT means recurrence of menopausal symptoms.

Density of estrogen receptors is highest in endometrium and vagina. A drop in estrogen levels results in
- a decrease in mitotic activity, so the vaginal lining becomes thinner and more fragile;
- a drop in collagen content in the connective tissue;
- decreased blood flow and decreased vaginal lubrication;
leading to vaginal symptoms and findings of pallor, dryness and decreased rugosity of the vaginal mucosa.

US 2003/0170325 A1 discloses compositions in gel or suppository form for the treatment of vaginal dryness. The compositions comprise herbal compounds with or without vitamin. However, this patent application does not suggest using extracts of myrrh for the treatment of vaginal dryness.

CN 1 679 687 A discloses a Chinese medicine in the form of externally applied capsules for treating vaginitis, cervical erosion, endometritis, hysteromyoma and adnexitis. This Chinese medicine is prepared from seven substances of traditional Chinese medicine, including cnidium fruit, myrrh, frankincense and dragon's blood.

WO 98/42188 A1 discloses a medical composition for use in treating diseases like HIV and other infections, wherein sesquiterpenes, vitamins and/or myrrh can be used as additive in connection to antimicrobial / antiviral plant extracts.

CN 101 057 892 A discloses a pharmaceutical composition comprising phellodendron, rhubarb, salvia miltiorrhiza, frankincense, myrrh, lithospermum, clam meal, and borneol as active ingredients. This pharmaceutical composition is intended to be used, amongst others, against vaginal inflammation.

CN 1 559 586 A discloses a composition of 15 substances of traditional Chinese medicine including mint, Chinese angelica and astragalus motherwort. This composition is intended for treating acute or chronic inflammatory gynopathy and cervical erosion.

ZA 2000/0192 A discloses a composition comprising myrrh, frankincense and gold. This composition is intended to be used for the treatment or prevention of physical and/or spiritual disorders in humans.

A non-hormonal effective treatment of menopausal symptoms is needed, as an alternative to HRT. It is an objective of the instant invention to provide an active ingredient for a pharmaceutical preparation or a medicinal product for such a treatment.

This objective is achieved by using myrrh in vaginal dosage form for the treatment of vaginal atrophy as menopausal symptom according to claim 1. In other words, the myrrh is used for the treatment of vaginal atrophy and symptoms related therewith occurring (not exclusively but also) during menopause. In the patent and non-patent literature known to the inventor, no teaching or suggestion for such an indication of myrrh could be found. This novel indication is surprising since vaginal atrophy as menopausal symptom is not related to bacteria and fungi against which myrrh has been used hitherto.

The term "myrrh" is to be understood as the oleo-gum resin originating from Commiphora species with the exception of Commiphora mukul. The myrrh used in connection to this invention is preferably pharmacopeia myrrh.

The menopausal symptom of vaginal atrophy manifests as vaginal dryness, itching, burning, and discomfort.

In an embodiment, the myrrh is present in the form of a powdered resin, a myrrh tincture or an extract of myrrh.

In another embodiment, the extract of myrrh is a dry extract (preferably obtained by extraction of myrrh with an organic solvent) or an aqueous extract. In a further embodiment, the myrrh is used in pastry form. To obtain a pastry, myrrh resin is subjected to extraction with an organic solvent, for example ethanol, petroleum ether or ethyl acetate. The extract thus obtained is filtered and vacuum-concentrated to a pastry form. Thus, myrrh pastry is a filtered and concentrated form of a myrrh extract. The residue is again dissolved in alcohol (e.g., ethanol) or a mixture of alcohol and water, filtered and vacuum-concentrated.

In a further embodiment, the myrrh is used as lipophilic extract of myrrh, in particular obtained by extraction with liquid carbon dioxide in the supercritical state. Other standard extraction methods as explained above are also applicable.

In a further embodiment, the extract contains furanoeudesma-1,3-diene, lindestrene and curzerene as main ingredients. The according chemical structures of these compounds are displayed below:

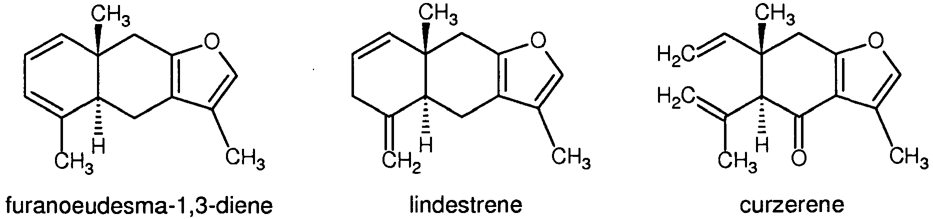

Preferably, furanoeudesma-1,3-diene is present in the extract in an amount of more than 20 %, in particular more than 25 %, in particular more than 30 % and very particular more than 35 %. All percentages given in the present application are to be understood as percent by weight if not explicitly indicated otherwise.

In a further embodiment, the extract contains more than 10 %, in particular more than 15 %, in particular more than 20 % and in particular more than 25 % of curzerene.

In a further embodiment, the extract contains more than 5 %, in particular more than 10 %, in particular more than 15 % of lindestrene.

In a further embodiment, the extract has a refractive index at 20 °C (measured with an Abbe refractometer) of 1.5 to 1.6, in particular of 1.51 to 1.55, in particular of 1.515 to 1.54, in particular of 1.517 to 1.537.

In a further embodiment, the extract has a density at 20 °C (measured with a pycnometer) of 1.0 to 2.0 g/cm³, in particular of 1.02 to 1.08 g/cm³, in particular of 1.025 to 1.075 g/cm³.

In a further embodiment, the myrrh is present in a semisolid dosage form for vaginal application, in particular as vaginal ointment, vaginal cream, or vaginal gel. In such semisolid preparations, oleaginous bases or water-soluble bases can be used. The semisolid preparations might be water-in-oil emulsions or oil-in-water emulsions.

In yet another embodiment, the myrrh is present in a liquid dosage form for vaginal application, in particular as vaginal liquid, vaginal emulsion, or vaginal suspension.

In another embodiment, the myrrh is present as vaginal foam or vaginal tampon.

In still another embodiment, the myrrh is present in a solid dosage form for vaginal application, in particular as vaginal tablet, vaginal capsule, or vaginal suppository.

Any of the afore-mentioned vaginal dosage forms enables a user to apply the myrrh at the location at which it can develop its activity against menopausal symptoms in a very well suited manner.

In a further embodiment, the solid dosage form, in particular the vaginal tablet, vaginal capsule, or vaginal suppository essentially consists of a fatty, oleaginous, or oil-type base and the myrrh. Cocoa butter and hard fat are members of this group of substances. Cocoa butter is an oleaginous base that softens at 30 °C and melts at about 34 °C, just below body temperature, thus representing an ideal suppository base. Other bases in this category include commercial products such as Fattibase (triglycerides from palm, palm kernel, and coconut oils with self-emulsifying glyceryl monostearate and polyoxyl stearate), Wecobee bases (triglycerides derived from coconut oil), Witepsol bases (triglycerides of saturated C₁₂-C₁₈ fatty acids with varying portions of the corresponding partial glycerides), Suppocire and Ovucire bases)

The inventor could already establish a synergistic effect of the combination of cocoa butter and myrrh. In particular, experiments indicate that constituents of cocoa butter and ingredients of myrrh essential oil show synergistic effects on mitotic activity, collagen metabolism, blood flow or lubrication when applied vaginally for treatment of vaginal atrophy. Thus, using cocoa butter as basic material even enhances the effectivity of the myrrh.

It was found that a very well suited dosage for using the myrrh, in particular in form of a suppository, is daily, in particular once a day, in particular once a day at bed time. Such a dosage regime is sufficient to alleviate the menopausal symptoms, in particular vaginal atrophy.

In an embodiment, the tablet, capsule or suppository has a weight of 0.5 to 5 g, in particular of 1 to 4 g, in particular of 2 to 3 g, in particular of 1.5 to 2.5 g, in particular of 1.7 to 1.9 g. 1.8 g is a particular well suited weight. Thereby, the myrrh is preferably present in the tablet, capsule or suppository in an amount of 0.1 to 10 %, in particular of 0.25 to 9 %, in particular of 0.5 to 8 %, in particular of 0.75 to 7 %, in particular of 1 to 6 %, in particular of 1.5 to 5 %, in particular of 2 to 4 %, in particular of 2.5 to 3 % based on the total weight of the tablet, capsule or suppository. An amount around 1 % of myrrh is particularly well suited.

Furthermore, a method for treating the (female) human or animal body with myrrh to relieve menopausal symptoms, in particular to relieve vaginal atrophy, is hereby disclosed. Explained embodiments of the claimed use of myrrh or the claimed pharmaceutical preparation are also applicable with respect to this method.

Further details of the instant invention are explained in connection to the following examples which are, however, not to be construed in a limiting way.

A lipophilic extract of Commiphora myrrha, produced by supercritical CO₂ extraction, was used.

Vaginal suppositories were prepared using either hard fat or cocoa butter as suppository base.

Cocoa butter was melted at 50 °C. Under stirring, myrrh extract was added to a final concentration of 1 % by weight of the total weight of the formula (mixture). Stirring was continued for several hours to obtain a stable crystalline form. The mixture was then poured into molds and cooled.

Hard fat was melted at 50 °C. Myrrh extract was added to a final concentration of 1% of the total weight of the formula to obtain a mixture. The mixture was poured into molds and cooled.

The suppositories weighed 2 grams each, containing 20 mg of myrrh extract each.

Both preparations (hard fat/cocoa butter) were tested in postmenopausal women (more than 2 years past last menstrual period) with vaginal symptoms of dryness, discomfort and itching. The vaginal suppositories were applied over a period of 14 consecutive days once a day, namely at bedtime each night.

Both preparations provided subjective and objective relief of menopausal symptoms, in particular vaginal symptoms. Colposcopy revealed an improved status of the vaginal mucosa.

Improvement of genital symptoms was more pronounced with the cocoa butter suppositories. Theobroma cocoa seeds contain polyphenols and flavonoids - substances with high antioxidation potential - and procyanidins which exhibit vascular effects. It appeared that in connection to the myrrh extract synergistic effects on mitotic activity, collagen metabolism, blood flow or lubrication occur when both substances are applied concomitantly for the treatment of vaginal atrophy.

## Claims

1. Myrrh in vaginal dosage form for use in the treatment of vaginal atrophy as menopausal symptom.

2. Myrrh for use according to claim 1, **characterized in that** the myrrh is present in the form of a powdered resin, a myrrh tincture or an extract of myrrh.

3. Myrrh for use according to claim 2, **characterized in that** the extract of myrrh is an aqueous extract, a dry extract, or a lipophilic extract.

4. Myrrh for use according to claim 2 or 3, **characterized in that** the myrrh tincture or the extract of myrrh contains furanoeudesma-1,3-diene, lindestrene and curzerene as main ingredients.

5. Myrrh for use according to any of the preceding claims, **characterized in that** it is present in a semisolid vaginal dosage form, in particular as ointment, cream, or gel.

6. Myrrh for use according to any of claims 1 to 4, **characterized in that** it is present in a liquid vaginal dosage form, in particular as liquid, emulsion, or suspension.

7. Myrrh for use according to any of claims 1 to 4, **characterized in that** it is present as vaginal foam or vaginal tampon.

8. Myrrh for use according to any of claims 1 to 4, **characterized in that** it is present in a solid vaginal dosage form, in particular as tablet, capsule, or suppository.

9. Myrrh for use according to any of the preceding claims, **characterized in that** it is to be used daily, in particular once a day.

## Patentansprüche

1. Myrrhe in vaginaler Darreichungsform zur Verwendung bei der Behandlung von Vaginalatrophie als menopausalem Symptom.

2. Myrrhe zur Verwendung gemäß Anspruch 1, **dadurch gekennzeichnet, dass** die Myrrhe in Form eines pulverisierten Harzes, einer Myrrhetinktur eines Myrrheextrakts vorliegt.

3. Myrrhe zur Verwendung gemäß Anspruch 2, **dadurch gekennzeichnet, dass** der Myrrheextrakt ein wässriger Extrakt, ein trockener Extrakt oder ein lipophiler Extrakt ist.

4. Myrrhe zur Verwendung gemäß Anspruch 2 oder 3, **dadurch gekennzeichnet, dass** die Myrrhetinktur oder der Myrrheextrakt Furaneudesma-1,3-dien, Lindestren und Curzeren als Hauptbestandteile enthält.

5. Myrrhe zur Verwendung gemäß einem der vorherigen Ansprüche, **dadurch gekennzeichnet, dass** sie in einer halbfesten vaginalen Darreichungsform vorliegt, insbesondere als Salbe, Creme oder Gel.

6. Myrrhe zur Verwendung gemäß einem der Ansprüche 1 bis 4, **dadurch gekennzeichnet, dass** sie in einer flüssigen vaginalen Darreichungsform vorliegt, insbesondere als Flüssigkeit, Emulsion oder Suspension.

7. Myrrhe zur Verwendung gemäß einem der Ansprüche 1 bis 4, **dadurch gekennzeichnet, dass** sie als Vaginalschaum oder Vaginaltampon vorliegt.

8. Myrrhe zur Verwendung gemäß einem der Ansprüche 1 bis 4, **dadurch gekennzeichnet, dass** sie in einer festen vaginalen Darreichungsform vorliegt, insbesondere als Tablette, Kapsel oder Zäpfchen.

9. Myrrhe zur Verwendung gemäß einem der vorherigen Ansprüche, **dadurch gekennzeichnet, dass** sie zur täglichen Anwendung vorgesehen ist, insbesondere einmal pro Tag.

## Revendications

1. Myrrhe sous forme de dosage par voie vaginale pour utilisation dans le traitement de l'atrophie vaginale comme un symptôme de ménopause.

2. Myrrhe pour utilisation selon la revendication 1, **caractérisé en ce que** la myrrhe est présent sous la forme d'une résine en poudre, d'une teinture de myrrhe ou d'un extrait de myrrhe.

3. Myrrhe pour utilisation selon la revendication 2, **caractérisé en ce que** l'extrait de myrrhe est un extrait aqueux, un extrait sec ou un extrait lipophile.

4. Myrrhe pour utilisation selon les revendications 2 ou 3, **caractérisé en ce que** la teinture de myrrhe ou l'extrait de myrrhe contient furanoeudesma-1,3-diène, lindestrène et curzérène comme ingrédients principaux.

5. Myrrhe pour utilisation selon l'une quelconque des revendications précédentes, **caractérisé en ce qu'**elle est présente sous une forme de dosage par voie vaginale semi-solide, en particulier sous forme de pommade, de crème ou de gel.

6. Myrrhe pour utilisation selon l'une quelconque des revendications 1 à 4, **caractérisé en ce qu'**elle est présente sous une forme de dosage par voie vaginale liquide, en particulier sous forme de liquide, d'émulsion, ou de suspension.

7. Myrrhe pour utilisation selon l'une quelconque des revendications 1 à 4, **caractérisé en ce qu'**elle est présente sous forme de mousse vaginale ou de tampon vaginal.

8. Myrrhe pour utilisation selon l'une quelconque des revendications 1 à 4, **caractérisé en ce qu'**elle est présente sous une forme de dosage par voie vaginale solide, en particulier sous forme de comprimé, de capsule, ou de suppositoire.

9. Myrrhe pour utilisation selon l'une quelconque des revendications précédentes, **caractérisé en ce qu'**elle est prévue pour une utilisation quotidienne, en particulier une fois par jour.
